# EUROPEAN PATENT SPECIFICATION

(11) **EP 2 078 524 B1**
(45) Date of publication and mention of the grant of the patent: **31.08.2016**
(21) Application number: 07830104.1
(22) Date of filing: 18.10.2007
(51) Int. Cl.: A61K 31/343, A61K 9/70, A61K 47/10, A61K 47/14, A61P 25/24

(54) **ADHESIVE SKIN PATCH**
KLEBENDES HAUTPFLASTER
TIMBRE TRANSDERMIQUE ADHÉSIF

(30) Priority: 27.10.2006 JP 2006293070
(43) Date of publication of application: 15.07.2009
(73) Proprietor: Hisamitsu Pharmaceutical Co., Inc., Tosu-shi, Saga 841-0017 (JP)
(72) Inventor: SUZUKI, Makoto, Tsukuba-shi Ibaraki 305-0856 (JP); AMANO, Satoshi, Tsukuba-shi Ibaraki 305-0856 (JP); TATEISHI, Tetsuro, Tosu-shi Saga 841-0017 (JP); TAKEUCHI, Akio, Tsukuba-shi Ibaraki 305-0856 (JP)
(74) Representative: Grünecker Patent- und Rechtsanwälte PartG mbB
(86) International application number: PCT/JP2007/070370
(87) International publication number: WO 2008/050673

(56) References cited:
- WO-A2-2005/079756
- US-A1- 2002 192 302
- US-B1- 6 203 817

## Description

### Technical Field

The present invention relates to a plaster, and more specifically it relates to a citalopram-containing plaster wherein the abundance ratio of the S form is greater than the abundance ratio of the R form.

### Background Art

Citalopram is a selective serotonin reuptake inhibitor (SSRI), and it is used as a drug for amelioration of major depressive disorders, neurotic disorders, acute stress disorders, eating disorders and the like. It is usually administered orally.

Citalopram is known to exist as two optical isomers, the R form and S form. As disclosed in Patent documents 1 and 2, for example, only the S form((+) form) of the two optical isomers exhibits efficacy as a drug.

SSRIs such as citalopram may produce side-effects such as nausea, diarrhea and gastrointestinal disorders when administered orally to patients, which may lead to reduced compliance such as discontinuance of medication. Alternatives to oral administration have therefore been investigated in recent years. Patent document 3, for example, deals with transdermal administration of antidepressants such as SSRIs, and mentions citalopram as an example of an antidepressant drug.

However, transdermal administration of citalopram has been problematic in that it produces strong skin irritation as a side-effect.

It has been reported in Patent document 4 that drugs such as citalopram that elicit skin irritation when used in free form, exhibit lower skin irritation when used as salts.
[Patent document 1] Japanese Patent Public Inspection No. 2004-527551
[Patent document 2] Japanese Unexamined Patent Publication HEI No. 2-36177
[Patent document 3] U.S. Patent Application Publication No. 2002/0192302
[Patent document 4] U.S. Patent No. 6203817

US6203817 solves the problem of skin irritancy of transdermal patches.

### Disclosure of the Invention

### Problems to be Solved by the Invention

The present inventors have found, however, that skin irritation by citalopram is not satisfactorily reduced even by the methods mentioned above.

The present invention has been accomplished in light of these circumstances, and its object is to provide a citalopram-containing plaster with satisfactorily reduced skin irritation.

### Means for Solving the Problems

With the aim of achieving the object stated above, the present inventors conducted diligent research on skin irritation caused by citalopram and its salts and obtained the following surprising results.

Specifically, it has been believed in the prior art that the side-effect of skin irritation occurs only with the S form of citalopram which is the efficacious active form of the drug. However it was demonstrated that citalopram produces essentially the same skin irritation in its racemic mixture, S form and R form, as explained in the reference examples provided below.

The present inventors conducted further research based on this knowledge, and as a result it was found that the object stated above can be achieved by a plaster comprising a backing layer and a drug layer laminated on the backing layer, wherein the drug layer contains an adhesive base and at least one compound selected from the group consisting of citalopram and pharmaceutically acceptable salts thereof, and wherein the abundance ratio of the S form is greater than the abundance ratio of the R form and when the plaster comprises the excipients as defined in claim 1.

The invention therefore provides a citalopram-containing plaster with satisfactorily reduced skin irritation.

The abundance ratio of the S form and R form of citalopram and pharmaceutically acceptable salts thereof is preferably 98.5:1.5-100:0. This will help further reduce skin irritation by the plaster.

The total content of the citalopram and pharmaceutically acceptable salts thereof in the plaster of the invention is preferably 3-15 wt% based on the total weight of the drug layer.

If the total content of citalopram and pharmaceutically acceptable salts thereof is less than 3 wt%, the drug effect may not be as sufficiently exhibited as when it is within the range specified above. If the total content of citalopram and pharmaceutically acceptable salts thereof is greater than 15 wt%, on the other hand, the skin irritation-reducing effect may not be as sufficiently exhibited as when it is within the range specified above.

The pharmaceutically acceptable salt in the plaster of the invention is preferably at least one selected from the group consisting of oxalates, hydrobromides and hydrochlorides, with oxalates being more preferred. This will improve the stability of the drug components in the formulation.

The adhesive base in the plaster of the invention preferably contains at least one polymer selected from the group consisting of acrylic polymers and rubber-based polymers. This will result in a more powerful skin irritation-reducing effect.

The drug layer in the plaster of the invention preferably further comprises at least one absorption accelerator selected from the group consisting of C6-20 aliphatic alcohols, C6-20 aliphatic ethers, C6-20 fatty acids, C6-20 fatty acid esters, C6-20 fatty acid amides, glycerin, glycerin fatty acid esters, propyleneglycols, propyleneglycol fatty acid esters, polyethylene glycol and polyethyleneglycol fatty acid esters (which compounds may be saturated or unsaturated, straight-chain or branched, or with a cyclic structure). This will result in an even more powerful skin irritation-reducing effect.

### Effect of the Invention

According to the invention it is possible to provide a citalopram-containing plaster with satisfactorily reduced skin irritation, excellent percutaneous absorption and an adequate drug effect, as well as high safety.

### Brief Description of the Drawings

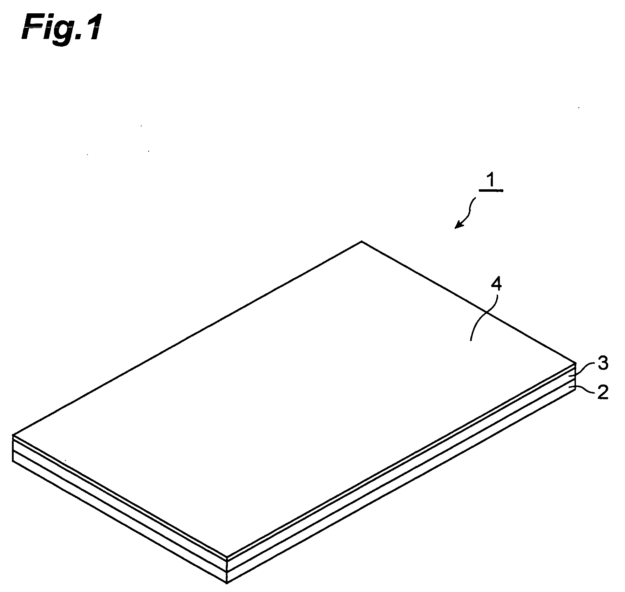

Fig. 1 is a perspective view of a preferred embodiment of the plaster of the invention.

### Explanation of Symbols

1: Plaster, 2: backing layer, 3: drug layer, 4: release liner.

### Best Modes for Carrying Out the Invention

Preferred embodiments of the plaster of the invention will now be described in detail.

The plaster of the invention comprises a backing layer and a drug layer laminated on the backing layer. The drug layer contains an adhesive base and at least one compound selected from the group consisting of citalopram and pharmaceutically acceptable salts thereof, wherein the abundance ratio of the S form is greater than the abundance ratio of the R form. The phrase "the abundance ratio of the S form is greater than the abundance ratio of the R form" means that the abundance ratio of the S form exceeds 50% for the citalopram in the drug layer.

According to the invention, the abundance ratio of the S form and R form for citalopram is preferably 70:30-100:0, more preferably 90:10-100:0, even more preferably 98.5:1.5-100:0 and most preferably 100:0. The skin irritation-reducing effect is improved with a smaller R form abundance ratio, and an R form abundance ratio of zero is encompassed within the scope of the invention.

There are no particular restrictions on the pharmaceutically acceptable salts, and as examples there may be mentioned inorganic acid salts such as hydrochlorides, sulfates, nitrates, phosphates and hydrobromides, and organic acid salts such as acetates, propionates, citrates, lactates, oxalates, succinates, tartrates, malonates, fumarates and malates. From the viewpoint of stability of the drug component in the formulation, oxalates, hydrobromides and hydrochlorides are preferred among those mentioned above, with oxalates being more preferred.

The drug layer may contain the citalopram and a pharmaceutically acceptable salt thereof each alone, or it may contain them in the form of a mixture. The total content of the citalopram and pharmaceutically acceptable salts thereof is preferably 3-15 wt% and more preferably 5-12.5 wt% based on the total weight of the drug layer.

Limiting the total content of citalopram and pharmaceutically acceptable salts thereof to within this range will allow a more satisfactory drug effect to be exhibited and produce a more notable skin irritation-reducing effect, than when the content is outside of the range.

The adhesive base is a rubber-based polymer. The rubber-based polymer is selected from styrene-isoprene-styrene block copolymer (hereinafter also referred to as "SIS"), isoprene rubber, polyisobutylene (hereinafter also referred to as "PIB"), styrene-butadiene-styrene block copolymer (hereinafter also referred to as "SBS"), styrene-butadiene rubber (hereinafter also referred to as "SBR"), polysiloxane and the like. SIS, PIB and polysiloxane are preferred among these, with SIS and PIB being especially preferred.

Such adhesive bases may be used alone or in combinations of two or more, with mixtures of SIS and PIB being especially preferred. This will improve the tack of the adhesive base and yield a plaster that exhibits higher adhesion.

The adhesive base content is preferably 5-90 wt% and more preferably 10-70 wt% based on the total weight of the drug layer. If the adhesive base content is within this range, the stability of the formed drug layer and the cutaneous permeability of the drug will be better than when it is outside of the range.

The drug layer may further contain additives such as absorption accelerators, organic acids, plasticizers, tackifiers, basic compounds and the like.

Absorption accelerators are selected from C6-20 aliphatic alcohols, C6-20 fatty acid esters, glycerin fatty acid esters, propyleneglycol. C6-20 fatty acid esters, C6-20 aliphatic alcohols and propylene glycols are more preferred, and isopropyl myristate and myristyl alcohol are especially preferred.

Such absorption accelerators may be used alone or in mixtures of two or more, and preferably myristyl alcohol and isopropyl myristate are used in admixture. By using a mixture of myristyl alcohol and isopropyl myristate, skin irritation is further reduced and percutaneous absorption is increased while maintaining formulation cohesion and adhesion.

The absorption accelerator content is preferably 0.01-40 wt% and more preferably 0.05-15 wt% based on the total weight of the drug layer. Limiting the absorption accelerator content to within this range will improve the cutaneous permeability of the drug while also reducing irritation to the skin such as redness and edema, compared to when the content is outside of the range.

Plasticizers are not particularly restricted so long as they are compounds with plasticity, and as examples there may be mentioned petroleum-based oils, (for example, paraffin-based process oils, naphthene-based process oils and aromatic process oils), squalane, squalene, vegetable oils (for example, olive oil, camellia oil, castor oil, tall oil and peanut oil), silicon oil, dibasic acid esters (for example, dibutyl phthalate and dioctyl phthalate), liquid rubbers (for example, polybutene, liquid isoprene rubber), liquid fatty acid esters (isopropyl myristate, hexyl laurate, diethyl sebacate, diisopropyl sebacate), diethylene glycol, polyethylene glycol, glycol salicylate, propylene glycol, dipropylene glycol, triacetin, triethyl citrate and crotamiton. Preferred among these are liquid paraffin, liquid polybutene, isopropyl myristate, diethyl sebacate and hexyl laurate, with liquid paraffin being particularly preferred.

Any of these plasticizers may be used alone or in mixtures of two or more. The content of the plasticizer is preferably 10-70 wt%, more preferably 10-60 wt% and even more preferably 10-50 wt% based on the total weight of the drug layer. Limiting the plasticizer content to within this range will improve the cutaneous permeability of the drug while also increasing the cohesion of the plaster, compared to when the content is outside of the range.

When the adhesive force of the drug layer is insufficient, it is preferred to add a tackifier. Tackifiers are not particularly restricted, and as examples there may be mentioned rosin derivatives (for example, rosin, rosin glycerin ester, hydrogenated rosin, hydrogenated rosin glycerin esters, rosin pentaerythritol ester and the like), alicyclic saturated hydrocarbon resins (for example, ARKON P100 by Arakawa Chemical Industries, Ltd.), aliphatic hydrocarbon resins (for example, QUINTONE B170 by Zeon Corp.), terpene resins (for example, CLEARON P-125 by Yasuhara Chemical Co., Ltd.) and maleic acid resins. Particularly preferred among these are hydrogenated rosin glycerin esters, alicyclic saturated hydrocarbon resins, aliphatic hydrocarbon resins and terpene resins.

Any of these tackifiers may be used alone or in mixtures of two or more. The content of the tackifier is preferably 5-70 wt%, more preferably 5-60 wt% and even more preferably 10-50 wt% based on the total weight of the drug layer. If the tackifier content is less than 5 wt%, the adhesive force of the plaster will tend to be lower than when it is within the aforementioned range. If the tackifier content exceeds 70 wt%, irritation to the skin during peeling will tend to be stronger than when it is within the aforementioned range.

When an acid addition salt is used as the pharmaceutically acceptable salt, the drug layer preferably contains a basic compound. As examples of basic compounds there may be mentioned low molecular compounds containing basic nitrogen (for example, triethanolamine, diisopropanolamine and diethanolamine), high molecular compounds containing basic nitrogen (for example, aminoalkyl methacrylate copolymer E, polyvinylacetal diethylaminoacetate and polyvinylpyridine), basic alkali metal salts (for example, sodium acetate, potassium acetate, sodium borate, sodium carbonate, trisodium citrate and sodium silicate), sodium hydroxide, or potassium hydroxide. Preferred among these are triethanolamine, diisopropanolamine, diethanolamine, aminoalkyl methacrylate copolymer E, polyvinylacetal diethylaminoacetate, sodium acetate, sodium silicate and sodium hydroxide, and especially triethanolamine, aminoalkyl methacrylate copolymer E, sodium acetate and sodium hydroxide.

Any of these basic compounds may be used alone or in mixtures of two or more. The basic compound content is preferably 0.5-3 equivalents and more preferably 1-2 equivalents with respect to the acid addition salt of citalopram. Also, the basic compound content is preferably 0.1-10 wt%, more preferably 1-9 wt% and even more preferably 1-7 wt% based on the total weight of the drug layer. If such a basic compound is included, the basic compound will act on the pharmaceutically acceptable acid addition salt of citalopram, thus improving the cutaneous permeability of the citalopram salt. A basic compound content within the aforementioned range will particularly provide a better effect of improving the cutaneous permeability than when it is outside of the range.

The plaster of the invention may be produced by a conventional method such as a solvent method or hot-melt method. When it is produced by a solvent method, for example, an organic solvent solution of the formulating composition stirred with other added components may be spread onto a backing layer and dried to form a drug layer, to obtain a plaster according to the invention. When the formulating composition is of a type that can be coated by a hot-melt method, the composition may be dissolved at high temperature and then spread onto a backing layer to form a drug layer, to obtain a plaster according to the invention.

The solvent used for production by a solvent method may be, for example, a production solvent such as a lower alcohol, toluene, ethyl acetate, hexane or cyclohexane, or the compound used as the plasticizer of the formulation. Preferred are methanol, ethanol, isopropanol, toluene, ethyl acetate and cyclohexane, with methanol, ethanol, toluene and ethyl acetate being particularly preferred.

The plaster of the invention may also be obtained by using a release liner (described hereunder) instead of a backing layer to form the drug layer, and then attaching a backing layer.

So long as the plaster of the invention has a drug layer with the composition described above and the backing layer is able to provide sufficient support, there are no particular restrictions on the other layers or the components composing them. For example, the plaster of the invention may contain a release liner on the drug layer, in addition to the backing layer and drug layer. The plaster of the invention may also comprise two or more layers containing adhesive bases, so long as the effect of the invention is not reduced.

The backing layer is not particularly restricted so long as it is suitable for supporting the drug layer, and it may be either elastic or non-elastic. As specific examples there may be mentioned cloths, nonwoven fabrics, polyurethane, polyester, polyvinyl acetate, polyvinylidene chloride, polyethylene, polyethylene terephthalate, aluminum sheets and the like, as well as composites of the foregoing. When the plaster of the invention is to be attached for prolonged periods (for example, 24 hours or longer), a cover material may be used if necessary to cover the plaster and prevent peeling.

The release liner is not particularly restricted so long as it has a sufficient release property from the drug layer, and polyethylene terephthalate (PET) films, polyethylene films, polypropylene films, polytetrafluoroethylene films and the like may be suitably used. Silicone-treated release liners are preferred.

Fig. 1 is a perspective view of a preferred embodiment of a plaster according to the invention as described above. The plaster 1 in Fig. 1 comprises a sheet-like backing layer 2, a drug layer 3 laminated on one side of the backing layer 2, and a release liner 4 laminated on the side of the drug layer 3 opposite the backing layer 2. The plaster 1 is used by peeling off the release liner 4 and then attaching the drug layer 3 onto the skin of a patient by adhesion.

The plaster of the invention can be easily modified for the symptoms, age, body weight and gender of a patient by, for example, cutting the plaster for a suitable dosage of the active drug. The area of the drug layer in the plaster of the invention that contacts the skin is not particularly restricted, but it is preferably 5-50 cm² and more preferably 5-30 cm². The area of the drug layer in the plaster that contacts the skin is less than 50 cm² in order to facilitate handling during application, while it is at least 5 cm² in order to help maintain sufficient cutaneous permeability for the drug.

### Examples

The present invention will now be explained in greater detail based on examples and comparative examples, with the understanding that the invention is in no way limited to the examples.

### (Reference example)

The degrees of skin irritation by escitalopram (S form of citalopram), racemic mixture of citalopram and the R form of citalopram were examined by a rabbit skin primary irritation test. Physiological saline solutions containing 0.1% and 0.3% of each compound were prepared and intradermally administered in the dorsal regions of the rabbits. The skin irritation was observed based on the Draize scale, and the skin irritation was evaluated as the score (Primary Irritation Index, P.I.I.) (n = 4). The results are shown in Table 1.

**[Table 1]**

| Sample | | Skin irritation score (P.I.I.) |
|---|---|---|
| Saline | | 0.1 |
| 0.1 % Saline solution | S form | 0.4 |
| | Racemic | 0.4 |
| | R form | 0.4 |
| 0.3% Saline solution | S form | 2.6 |
| | Racemic | 2.5 |
| | R form | 2.6 |

The results clearly show essentially no difference in irritation between the S form, racemic mixture and R form of citalopram, and therefore all of the compounds have approximately the same skin irritability.

### (Example 1)

Escitalopram (S form of citalopram, Zhejiang Haisen Pharmaceutical Co., Ltd., 98.5+% purity), isopropyl myristate, liquid paraffin and myristyl alcohol were thoroughly mixed. To the obtained mixture there was added a mixture of styrene-isoprene-styrene block copolymer (SIS5002, product of JSR), polyisobutylene (Vistanex L-100:LM-MH = 1:3 (weight ratio), product of ExxonMobil), an alicyclic saturated hydrocarbon resin (ARKON P100, product of Arakawa Chemical Industries, Ltd.) and toluene, to prepare a drug layer coating solution. The obtained coating solution was then coated onto a polyethylene terephthalate release liner, and the solvent was removed by drying to form a drug layer. A polyester knitted fabric was attached as a backing layer onto the drug layer to obtain a plaster of Example 1. The weight proportions of the components are shown in Table 2.

### (Examples 2-12)

Plasters of Examples 2-12 were produced in the same manner as Example 1, except that the rubber-based polymers, drugs and additives were used in the weight proportions shown in Table 2.

### (Comparative Example 1)

A plaster of Comparative Example 1 was produced in the same manner as Example 1, except that the rubber-based polymer, drug and additives were used in the weight proportions shown in Table 2.

**[Table 2]**

| | | Example 1 | Example 2 | Example 3 | Example 4 | Example 5 | Example 6 | Example 7 | Example 8 | Example 9 | Example 10 | Example 11 | Example 12 | Comp. Ex. 1 |
|---|---|---|---|---|---|---|---|---|---|---|---|---|---|---|
| Rubber-based polymers | Styrene-isoprene-styrene copolymer | 17 | 16.7 | 16 | 15.6 | 15.2 | 15.2 | 15.2 | 15.2 | 15.2 | 15.2 | 15.2 | 15.2 | 12.8 |
| | polyisobutylene | 7.3 | 7.1 | 6.8 | 6.7 | 6.5 | 6.5 | 6.5 | 6.5 | 6.5 | 6.5 | 6.5 | 6.5 | 5.5 |
| Drugs | Citalopram hydrobromide | - | - | - | - | - | - | - | - | - | - | - | - | 15 |
| | Escitalopram I | 3.9 | 5.9 | 9.8 | - | - | - | - | - | - | - | - | - | - |
| | Escitalopram oxalate | - | - | - | 7.5 | 7.5 | 7.5 | 7.5 | 7.5 | 7.5 | 7.5 | 7.5 | 7.5 | - |
| Additives | Alicyclic hydrocarbon resin | 43.9 | 42.9 | 41.1 | 40 | 39 | 39 | 39 | 39 | 39 | 39 | 39 | 39 | 32.9 |
| | Liquid paraffin | 14.9 | 14.4 | 13.3 | 11.8 | 17.3 | 17.3 | 17.3 | 17.3 | 17.3 | 17.3 | 17.3 | 17.3 | 18.7 |
| | Pyrothiodecane | - | - | - | - | - | - | - | - | - | | - | - | 3 |
| | Isopropyl myristate | - | - | 10 | - | 10 | - | - | - | - | - | - | - | - |
| | Isopropyl palmitate | - | - | - | - | - | 10 | - | - | - | - | - | - | - |
| | oleate | - | - | - | - | - | - | 10 | - | - | - | - | - | - |
| | Glyceryl Methyl laurate | - | - | - | - | - | - | - | 10 | - | - | - | - | - |
| | Diisopropyl adipate | - | - | - | - | - | - | - | - | 10 | - | - | - | - |
| | Myristyl alcohol | - | - | 3 | - | - | 3 | - | - | - | 10 | - | - | 3 |
| | Octyldodecanol | - | - | - | - | - | - | - | - | - | - | 10 | - | - |
| | Isostearyl alcohol | - | - | - | - | - | - | - | - | - | - | - | 10 | - |
| | Propylene glycol | - | - | - | - | - | - | - | - | - | - | - | - | - |
| | Sodium acetate | - | - | - | 4.4 | 4.5 | 4.5 | 4.5 | 4.5 | 4.5 | 4.5 | 4.5 | 4.5 | 9.1 |

### (Example 13)

A solution of escitalopram oxalate, boric acid, light silicic anhydride (AEROSIL), sodium acetate, sorbitan monooleate and an acrylic polymer (acrylic acid ester-vinyl acetate copolymer, DuroTak87-2516, product of National Starch and Chemical Company) in an ethyl acetate-ethanol-heptane-methanol mixed solvent was mixed to prepare a coating solution. The obtained coating solution was then coated onto a polyethylene terephthalate release liner, and the solvent was removed by drying to form a drug layer. A polyester knitted fabric was attached onto the drug layer to obtain a plaster of Example 13. The weight proportions of the components are shown in Table 3.

### (Examples 14 and 15)

Plasters of Examples 14 and 15 were produced in the same manner as Example 13, except that the acrylic polymers, drugs and additives were used in the weight proportions shown in Table 3.

### (Comparative Example 2)

Citalopram hydrobromide, pyrothiodecane, isopropyl myristate, polyoxyethylene monolaurate, sodium acetate, an acrylic polymer (trade name: MatriDerm C) and ethyl acetate were mixed to prepare a coating solution. The obtained coating solution was then coated onto a polyethylene terephthalate release liner, and the solvent was removed by drying to form a drug layer. A polyester knitted fabric was attached onto the drug layer to obtain a plaster of Comparative Example 2. The weight proportions of the components are shown in Table 3.

**[Table 3]**

| | | Example 13 | Example 14 | Example 15 | Comp. Ex. 2 |
|---|---|---|---|---|---|
| Acrylic polymer | | 63.1 | 55.1 | 51.8 | 43.9 |
| Drugs | Citalopram hydrobromide | - | - | - | 15 |
| | Escitalopram | - | - | - | - |
| | Escitalopram oxalate | 10 | 15 | 15 | - |
| Additives | Pyrothiodecane | - | - | - | 3 |
| | Isopropyl myristate | - | - | - | 26 |
| | Sorbitan monooleate | 10 | 10 | 10 | - |
| | Polyoxyethylene monolaurate | - | - | - | 3 |
| | Light silicic anhydride | 6 | 6 | 6 | - |
| | Boric acid | 5 | 5 | 5 | - |
| | Sodium acetate | 5.9 | 8.9 | 8.9 | 9.1 |
| | Acetic acid | - | - | 3.3 | - |

### [Evaluation of plasters]

The plasters obtained in Examples 1-4 and 13-15 and Comparative Examples 1 and 2 were provided for a cutaneous permeability test and a skin primary irritation test.

### (Cutaneous permeability test)

First, skin was peeled from the back of a hairless mouse and fitted in a flow-through cell with exterior circulation of 32°C hot water, with the dermis side on the receptor tank side. Next, each of the plasters of Examples 1-4 and 13-15 and Comparative Examples 1 and 2 (drug layer applied area: 5 cm²) was attached to the horny layer side of the skin, and using phosphate buffer (pH 7.4) as the receptor layer, the receptor solution was sampled at 5 ml/hr every 2 hours for 24 hours and the flow rate was measured, while also measuring the drug concentration by high-performance liquid chromatography. The skin permeation rate of the drug per hour was calculated from the measured values, and the skin permeation rate of the drug per unit area of skin with steady state measurement was determined. Table 4 and Table 5 show the maximum skin permeation rates of the drugs from the start of the test until 24 hours thereafter (maximum skin permeation rates) and total permeated drug doses (skin permeated drug doses).

### (Skin primary irritation test)

The plasters of Examples 1-4 and 13-15 and Comparative Examples 1 and 2 (drug layer applied area: 4 cm²) were attached to the backs of rabbits (Japanese white rabbits) for 24 hours and then removed, and the skin irritation was observed 1, 24 and 48 hours thereafter based on the Draize scale, with evaluation as the skin irritation score (Primary Irritation Index, P.I.I.) (n = 6). The results are shown in Tables 4 and 5.

**[Table 4]**

| | Example 1 | Example 2 | Example 3 | Example 4 | Comp. Ex. 1 |
|---|---|---|---|---|---|
| Maximum skin permeation rate (µg/cm²/hr) | 19.6 | 26.4 | 41.1 | 27.6 | 25.2 |
| Cumulative skin permeated dose (µg/cm²) | 292.0 | 434.2 | 610.7 | 477.5 | 310.9 |
| Skin irritation score (P.I.I.) | 0.1 | 0.4 | 0.7 | 0.9 | 2.3 |

**[Table 5]**

| (reference examples) | | | | |
|---|---|---|---|---|
| | Example 13 | Example 14 | Example 15 | Comp. Ex. 2 |
| Maximum skin permeation rate (µg/cm²/hr) | 14.1 | 23.4 | 28.2 | 61.6 |
| Cumulative skin permeated dose (µg/cm²) | 252.5 | 384.8 | 468.7 | 995 |
| Skin irritation score (P.I.I.) | 2.3 | 2.8 | 2.7 | 3.6 |

As clearly shown by the results, the plasters of Examples 1-4 which employed a rubber-based polymer had lower skin irritation than the plaster of Comparative Example 1 which employed the same rubber-based polymer. Also, the plasters of Examples 13-15 which employed an acrylic polymer had lower skin irritation than the plaster of Comparative Example 2 which employed an acrylic polymer. As these results clearly demonstrate, a plaster containing the S form of citalopram produces less skin irritation than a plaster containing a racemic mixture of citalopram, when using either a rubber-based polymer or an acrylic polymer as the adhesive base.

Furthermore, even though citalopram has similar skin irritation as a racemic mixture and as the S form as mentioned above, the skin irritation scores were lower than the skin irritation scores predicted from the weight ratios of the drugs in Examples 1-4 and Comparative Example 1. This suggests that when the rubber-based polymer was used, the skin irritation was reduced by the effect of the additives such as isopropyl myristate.

Furthermore, considering that the cutaneous permeabilities of the S form and R form are approximately the same, a comparable effect should be obtainable when the skin permeated dosage using the S form of citalopram, which exhibits the drug effect, is 1/2 that using a racemic mixture of citalopram. It is believed that sufficient drug effects were obtained because the cumulative skin permeated doses with the plasters of Examples 1-4 were larger than 1/2 of the cumulative skin permeated dose with the plaster of Comparative Example 1.

## Claims

1. A plaster comprising a backing layer and a drug layer laminated on the backing layer,
wherein the drug layer contains an adhesive base and at least one compound selected from the group consisting of citalopram and pharmaceutically acceptable salts thereof, the abundance ratio of the S form being greater than the abundance ratio of the R form, and
wherein the adhesive base contains at least one rubber-based polymer selected from the group consisting of styrene-isoprene-styrene block copolymers, isoprene rubbers, polyisobutylene, styrene-butadiene-styrene block copolymers, styrene-butadiene rubbers and polysiloxane, and
wherein the drug layer further contains at least one absorption accelerator selected from the group consisting of C6-20 aliphatic alcohols, C6-20 fatty acid esters, glycerin fatty acid esters and propyleneglycol.

2. The plaster according to claim 1, wherein the abundance ratio of the S form and R form of citalopram and pharmaceutically acceptable salts thereof is 98.5:1.5-100:0.

3. The plaster according to claim 1, wherein the total content of the citalopram and pharmaceutically acceptable salts thereof is 3-15 wt% based on the total weight of the drug layer.

4. The plaster according to claim 1, wherein the pharmaceutically acceptable salt is at least one selected from the group consisting of oxalates, hydrobromides and hydrochlorides.

5. The plaster according to claim 1, wherein the pharmaceutically acceptable salt is an oxalate.

## Patentansprüche

1. Pflaster umfassend eine Trägerschicht und eine Arzneimittelschicht, die auf die Trägerschicht laminiert ist,
wobei die Arzneimittelschicht einen Klebstoffgrundstoff und wenigstens eine Verbindung, ausgewählt aus der Gruppe bestehend aus Citalopram und pharmazeutisch verträglichen Salzen davon, enthält, wobei das Häufigkeitsverhältnis der S-Form größer ist als das Häufigkeitsverhältnis der R-Form, und
wobei der Klebstoffgrundstoff wenigstens ein Polymer auf Kautschukbasis, ausgewählt aus der Gruppe bestehend aus Styrol-Isopren-Styrol-Blockcopolymeren, Isoprenkautschuken, Polyisobutylen, Styrol-Butadien-Styrol-Blockcopolymeren, Styrol-Butadien-Kautschuken und Polysiloxan, enthält, und
wobei die Arzneimittelschicht außerdem wenigstens einen Absorptionsbeschleuniger, ausgewählt aus der Gruppe bestehend aus aliphatischen C6-20-Alkoholen, C6-20-Fettsäureestern, Glycerin-fettsäureestern und Propylenglycol, enthält.

2. Pflaster gemäß Anspruch 1, wobei das Häufigkeitsverhältnis der S-Form und R-Form von Citalopram und pharmazeutisch verträglichen Salzen davon 98,5:1,5-100:0 beträgt.

3. Pflaster gemäß Anspruch 1, wobei der Gesamtgehalt des Citaloprams und der pharmazeutisch verträglichen Salze davon 3-15 Gew.-%, bezogen auf das Gesamtgewicht der Arzneimittelschicht, beträgt.

4. Pflaster gemäß Anspruch 1, wobei das pharmazeutisch verträgliche Salz wenigstens eines, ausgewählt aus der Gruppe bestehend aus Oxalaten, Hydrobromiden und Hydrochloriden, ist.

5. Pflaster gemäß Anspruch 1, wobei das pharmazeutisch verträgliche Salz ein Oxalat ist.

## Revendications

1. Emplâtre comprenant une couche dorsale et une couche médicamenteuse stratifiée sur la couche dorsale,
où la couche médicamenteuse contient une base adhésive et au moins un composé sélectionné dans le groupe constitué du citaloprame et de ses sels pharmaceutiquement acceptables, le rapport d'abondance de la forme S étant supérieur au rapport d'abondance de la forme R, et
où la base adhésive contient au moins un polymère à base de caoutchouc sélectionné dans le groupe constitué des copolymères séquencés de styrène-isoprène-styrène, des caoutchoucs d'isoprène, du polyisobutylène, des copolymères séquencés de styrène-butadiène-styrène, des caoutchoucs de styrène-butadiène et du polysiloxane, et
où la couche médicamenteuse contient en outre au moins un accélérateur d'absorption sélectionné dans le groupe constitué des alcools aliphatiques en C₆ à C₂₀, des esters d'acide gras en C₆ à C₂₀, des esters d'acide gras de glycérine et du propylène glycol.

2. Emplâtre selon la revendication 1, où le rapport d'abondance de la forme S et de la forme R du citaloprame et de ses sels pharmaceutiquement acceptables est de 98,5:1,5 à 100:0.

3. Emplâtre selon la revendication 1, où la teneur totale du citaloprame et de ses sels pharmaceutiquement acceptables est de 3 à 15 % en pds sur la base du poids total de la couche médicamenteuse.

4. Emplâtre selon la revendication 1, où le sel pharmaceutiquement acceptable est au moins l'un sélectionné dans le groupe constitué des oxalates, des bromhydrates et des chlorhydrates.

5. Emplâtre selon la revendication 1, où le sel pharmaceutiquement acceptable est un oxalate.
